# EUROPEAN PATENT SPECIFICATION

(11) **EP 1 739 585 B1**
(45) Date of publication and mention of the grant of the patent: **10.03.2010**
(21) Application number: 06253142.1
(22) Date of filing: 16.06.2006
(51) Int. Cl.: G06F 19/00, A61M 5/142, A61M 5/168, A61M 5/172, G01N 21/00

(54) **Pre-delivery drug identification method**
Verfahren zur Identifizierung von Medikamenten, vor Verabreichung
Procédé d'identification de médicaments pré-livrés

(30) Priority: 21.06.2005 US 157415
(43) Date of publication of application: 03.01.2007
(73) Proprietor: GENERAL ELECTRIC COMPANY, Schenectady, NY 12345 (US)
(72) Inventor: Sandy, Neal Joseph, Middleton WI 53562 (US); Makin, Ronald Peter, Fitchburg WI 53711 (US); Ward, Russel Craig, Sun Prairie WI 53590 (US); Sutherland, William Scott, Haverhill MA 01830 (US)
(74) Representative: Illingworth-Law, William Illingworth

(56) References cited:
- WO-A-2005/046766
- US-A- 5 153 827
- US-A1- 2003 204 330
- US-A1- 2004 176 984
- US-E1- R E38 189

## Description

The invention relates to the field of drug delivery. More particularly, the invention relates to the field of identifying and confirming drug dosages before administering them to a patient.

Preventable medical errors are becoming increasingly prevalent in the health care industry. Among the biggest problems is adverse drug events or delivering a drug to a patient that should not be given to that patient.

A November 1999 institute of Medicine report states that "The medication process provides an example where implementing better systems will yield better human performance. Medication errors now occur frequently in hospitals, yet many hospitals are not making use of known systems, nor are they actively pursing new safety systems."

WO 2005/046766 discloses a process of verifying pharmaceuticals in an infusion pumping system. A processor determines if a match or a mismatch exists between an expected drug spectral pattern of a drug library selection and the actual drug reading from a fluid infusion channel. If the processor were to determine that the drug detected in the fluid channel is inconsistent with the drug expected to be there, the processor initiates a signal to a pump to cease the infusion.

US 2003/0204330 discloses spectroscopic analysis of intravenous fluid components.

There has been significant effort in developing systems that are process focused such as bar coding and radiofrequency "tagging" of drugs. Since these methods are process focused, they are inherently prone to error, even though they are an improvement to systems without such processes. Only the identification of the drug itself will insure that the proper drug is being delivered to the patient.

The present invention is a method of pre-delivery drug identification. The method is implemented where drugs are being administered, such as in a hospital or clinic, and identifies the drug being administered to the patient before the drug reaches the patient. After identification, the method is configured to cross-reference the identified drug with the patient's prescription and allergy information, and to prevent delivery to the patient, if necessary.

The present invention is a method of pre-delivery drug identification which comprises receiving a drug sample from a drug source into a drug identification unit, identifying the drug sample with the drug identification unit, cross-referencing the identified drug sample with a set of databases, such that the set of databases are configured to confirm the identified drug sample is appropriate and/or safe for a patient; and preventing the identified drug sample from being administered to a patient with a delivery prevention unit when the set of databases does not confirm the identified drug sample is appropriate and/or safe for a patient, The drug sample may correspond to a prescription for the patient, and the identifying step may further include identifying the components, the concentration and the dosage of the drug sample. The set of databases may include, but is not limited to a prescription database, an allergy database, and a drug interaction database, and the databases may be central or locally located, The delivery prevention unit may be any of the following: a flow valve or an IV line restrictor device, and the drug source may be any of a volumetric pump, a syringe pump, or a manual infusion device such as, but not limited to, a bolus syringe.

The invention will now be described in greater detail, by way of example, with reference to the drawings, in which:-
Figure 1A-Figure 1D are block diagrams illustrating embodiments of the system of the present invention.
Figure 2 is a flow chart illustrating an embodiment of the method of the present invention.

The invention is a drug identification system that identifies the drug prior to delivery to a patient. The identification system identifies the compound and/or concentration, cross-references the identification with the prescription, allergies and interactions databases via an interfaced data management system, and has a means to prevent delivery to the patient. Once the appropriate drug is confirmed, the drug compound can be delivered to the patient. The system will record the compound, when it was given and by which clinician. The system would deploy a stop-cock or other line clamping mechanism in order to stop drug flow to the patient prior to actual delivery. The system may or may not have a means to enunciate or otherwise transmit information about the relative correctness of the drug delivery. The system would alert or alarm in a manner that informs the clinician of a potential adverse drug event. The systems will maintain a record of measured drugs and will allow for single or multiple lines to be analyzed.

The primary advantage of the invention is that it will minimize the number of adverse drug events that occur in hospitals, clinics and surgery centers. This advantage may also be translated to home use, emergency and ambulatory use and use in the military. Because the system is able to identify the concentration and/or the compound of the drug itself, it is the most accurate method of drug identification possible. Systems available today rely on processes that have varying levels of compliance and varying levels of propensity for error. And, since the system is dynamic and linked to a drug library via an information system (for example), it can not only detect the drug, but also determine if the drug should be delivered to the patient by comparing the drug to the prescription order as well as to the patient's condition, allergies, or other physiological information, as they are found on the patient's electronic medical record. The ability to automatically stop the delivery of the drug sets the invention apart from other identification systems as well.

Furthermore, the system's ability to determine drug concentrations is highly advantageous and unique. Because the identification system is in-line, yet non-invasive, the system does not waste any drug, and it measures the specific drug sample that is intended for the patient. An alternative embodiment of the present invention implements a split-stream configuration, wherein the sample from the drug source is routed away from the line, is tested, and does not re-enter the line. Finally, drug mixtures (cocktails) would be easily identified and could be disallowed by the system.

It should also be noted that all current drug identification methods are indirect, meaning the drug itself is not identified. This invention actually identifies the drug. Because the identification system can detect specific drug characteristics, it will also recognize mixtures, thereby allowing for the prevention of drug cocktails. All current drug identification methods do not have a means to determine drug concentrations. This invention has such a capability. All current methods cannot stop the inappropriate delivery of a drug. This invention has the ability to automatically stop the delivery of a drug, thereby preventing adverse drug events. The invention can apply to mechanical drug delivery equipment such as IV delivery devices (volumetric or syringe pump) as well as bolus delivery methods. The detection method is non-invasive to the compound which maintains the purity of the drug.

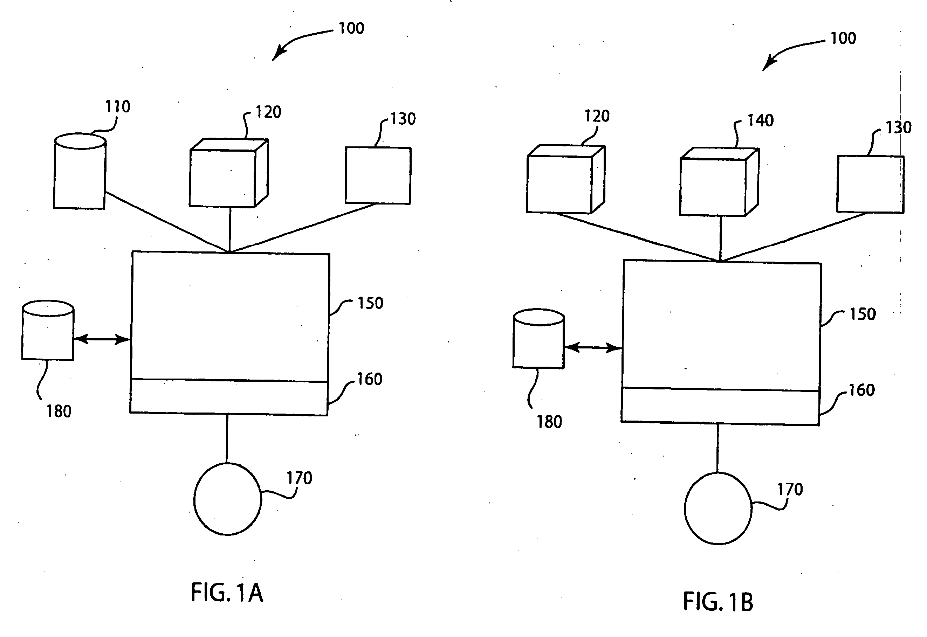

A drug identification system 100 of the present invention is depicted in Figures 1A-1D. In Figures 1A-1D, the drug identification system 100 includes a drug identification unit 150 configured to receive a sample of a drug to be administered to the patient 170 from a drug source 110, 120, 130, 140. Multiple embodiments of the present invention include various drug sources such as a volumetric pump 110, a syringe pump 120, 140, or a manual infusion device 130, such as, but not limited to a bolus syringe. It should be noted that the drug source 110, 120, 130, 140 utilized in any embodiment of the present invention is likely to be determined by the preference of the physician, the standard practice of the region and/or the particular drug or type of care being administered. Furthermore, referring to Figure 1B, particular embodiments may utilize more than one particular type of drug source such as a first syringe pump 120 and a second syringe pump 140. As is well known in the art, volumetric pumps 110 and syringe pumps 120, 140 are metered drug delivery sources, while a manual infusion device 130 is not of the metered variety.

Still referring to Figures 1A-1D, when a physician prescribes a drug to be administered to a patient 170, one of the aforementioned drug sources 110, 120, 130, 140 is utilized to deliver the drug to the patient 170. The drug identification system 100 of the present invention contemplates receiving a drug sample from the drug source 110, 120, 130, 140 into a drug identification unit 150. The drug identification unit 150 is able to test the drug sample from the drug source 110, 120, 130, 140 and determine a number of characteristics of the drug sample. The drug identification unit 150 is able to identify the element or compound of the drug sample, the concentration of the drug sample, as well as the dosage of the drug sample.

In the preferred embodiment of the present invention, the drug identification unit 150 utilizes raman scattering spectroscopy as a technique to analyze and identify the drug to be administered to the patient 170. The details of raman scattering spectroscopy are included in United States Patent No. 6,868,344 to Nelson. It is further contemplated that alternative embodiments of the present invention can and will include a drug identification unit 150 utilizing other methods known in the art, or future drug identification methods.

The drug identification unit 150 is coupled to a set of patient databases 180. The drug identification unit 150 cross-references the set of patient databases 180 in order to confirm that the drug sample delivered by the drug source 110, 120, 130, 140 is appropriate and safe for the patient 170. The set of databases 180 includes a prescription database, an allergy database and an interaction database, such that the identified drug sample can be cross-referenced against each of these databases 180. Of course, additional databases can be added or subtracted to the set of patient databases 180 as needed or required. Furthermore, the databases may be configured centrally in a network configuration, or locally to the drug identification system 100. In fact, any database 180 configuration known in the art may be used according to the constraints of the particular drug identification system 100.

The drug identification unit 150 will cross-reference the identified drug sample to determine whether the drug sample matches the prescription written by the physician, and will further cross-reference whether the patient 170 is allergic to the drug sample, and further whether the drug sample would interact with any of the patient's 170 other medications. As stated previously, the drug identification unit 150 is configured to identify not only what the drug sample is made up of, but also the concentration and the dosage of the drug sample, thus allowing the drug identification unit 150 to exactly match the drug sample to the prescription.

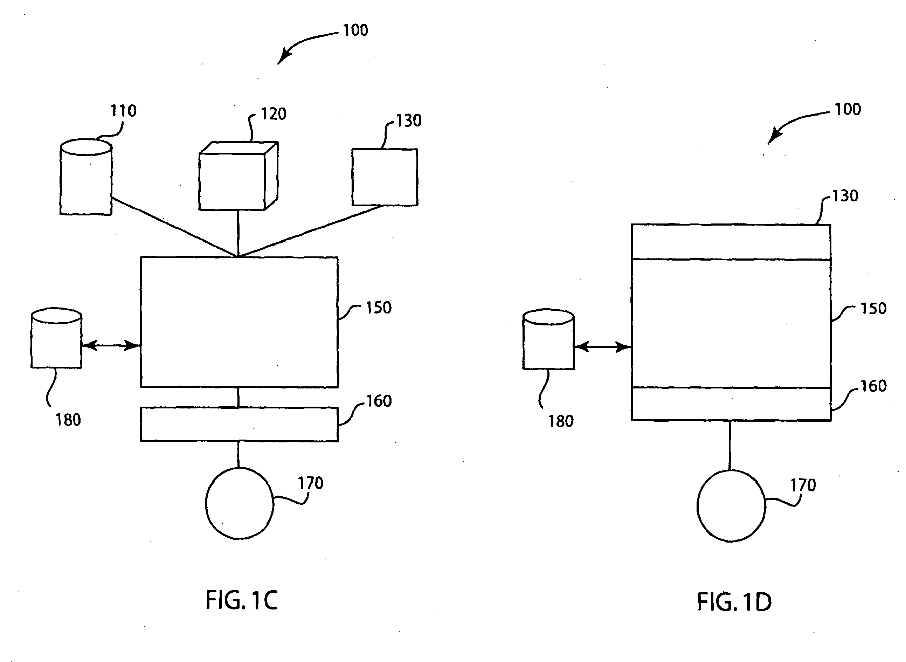

Still referring to Figures 1A-1D, the drug identification system 100 of the present invention also includes a delivery prevention unit 160. If the drug identification unit 150, when cross-referencing the identified drug sample to the set of patient databases 180, cannot confirm that the identified drug sample is appropriate and/or safe to administer to the patient 170, the delivery prevention unit 160 will prevent the drug sources 110, 120, 130, 140 from administering the drug to the patient 170. Preferably, the delivery prevention unit 160 is an electronically controlled flow valve, but can also be any IV flow restrictor known or later developed in the art. Only when the drug identification unit 150 is able to cross-reference the set of patient databases 180, and confirm that the drug sample exactly matches the prescription, and is not harmful to the patient, will the drug identification unit prompt the delivery prevention unit 160 to allow the drug to be administered to the patient 170. As is shown in Figures 1A, 1B and 1D, preferably the delivery prevention unit 160 is directly coupled to the drug identification unit and is likely implemented with the drug identification unit. Other embodiments, such as that depicted Figure 1C, will include a delivery prevention unit 160 separate yet coupled to the drug identification unit 150. Referring to Figure 1D, the drug identification system 100 of the present invention may also be implemented in one unit, including a manual infusion device 130, a drug identification unit 150 and a delivery prevention unit 160.

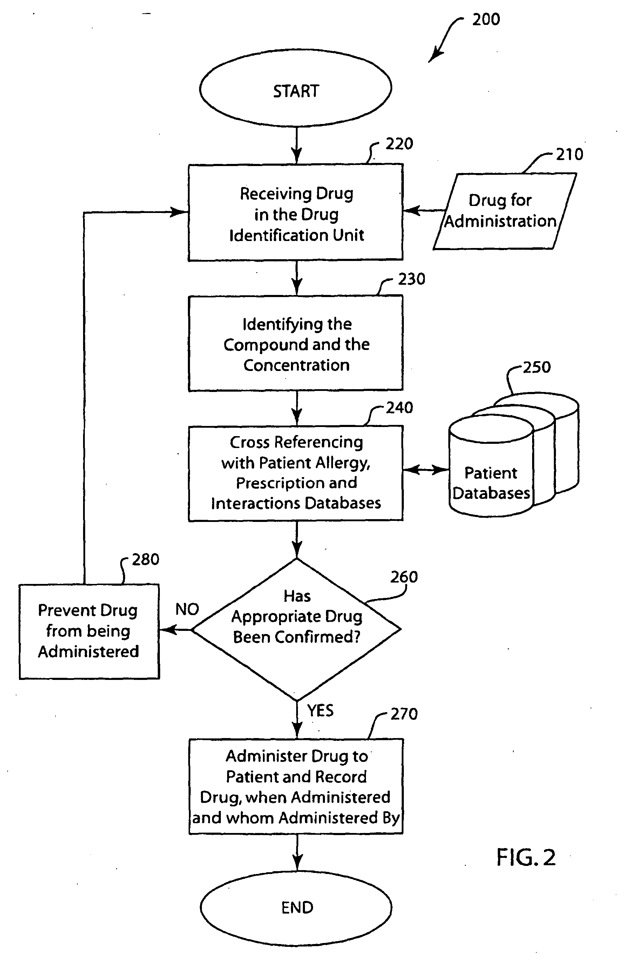

Referring now to Figure 2, a drug identification method 200 of the present invention is depicted. In step 220, the drug for administration 210 to the patient is received in the drug identification unit. In step 230, the drug is identified, including the actual compound, the concentration and the dosage of the drug for administration 210. In step 240, the identified drug is cross-referenced with a set of patient databases 250 to confirm whether the identified drug matches the prescription written by the physician. In step 240, the identified drug is also cross-referenced to the patient databases 150 to determine whether the identified drug will cause problems with the patients due to allergies and/or interactions with other drugs. In step 260, if the identified drug is not confirmed as the proper prescription or being dangerous to the patient, then in step 280 the identified drug is prevented from being administered to the patient and the method 200 starts again at step 220. However, if the identified drug is confirmed to be appropriate for administration to the patient, then the drug is administered to the patient in step 270. Furthermore, in step 270, the administration of the identified drug is recorded, and the time and the person who administered the drug is recorded as well.

## Claims

1. A method of pre-delivery drug identification, the method comprising:
a.) receiving (220) a drug sample from a drug source into a drug identification unit;
b.) identifying (230) the drug sample with the drug identification unit;
c.) cross-referencing (240) the identified drug sample with a set of databases, such that the set of databases are configured to confirm the identified drug sample is appropriate and/or safe for a patient; and
d.) preventing (280) the identified drug sample from being administered to a patient with a delivery prevention unit (160) when the set of databases does not confirm the identified drug sample is appropriate and/or safe for a patient.

2. The method according to claim 1, wherein the drug sample corresponds to a prescription for the patient.

3. The method according to claim 1, wherein the identifying step further includes identifying the components, the concentration and the dosage of the drug sample.

4. The method according to claim 1, wherein the set of databases include:
a.) a prescription database;
b.) an allergy database; and
c.) a drug interaction database.

5. The method according to claim 1, wherein the delivery prevention unit is any of the following:
a.) a flow valve; and
b.) an IV line restrictor device.

6. The method according to claim 1, wherein the drug source is a volumetric pump.

7. The method according to claim 1, wherein the drug source is a syringe pump.

8. The method according to claim 1, wherein the drug source is a manual infusion device.

## Patentansprüche

1. Verfahren zur Identifizierung von Medikamenten vor ihrer Verabreichung, wobei das Verfahren die Schritte aufweist:
a.) Aufnehmen (220) einer Medikamentenprobe aus einer Medikamentenquelle in eine Medikamentenidentifikationseinheit;
b.) Identifizieren (230) der Medikamentenprobe mittels der Medikamentenidentifizierungseinheit;
c.) Quervergleichen (240) der identifizierten Medikamentenprobe mit einem Satz von Datenbanken dergestalt, dass der Satz von Datenbanken zur Bestätigung konfiguriert ist, dass die identifizierte Medikamentenprobe für einen Patienten geeignet und/oder sicher ist; und
d.) Verhindern (280) einer Verabreichung der identifizierten Medikamentenprobe an einen Patienten mittels einer Zuführungsverhinderungseinheit (160), wenn der Satz von Datenbanken nicht bestätigt, dass die identifizierte Medikamentenprobe für einen Patienten geeignet und/oder sicher ist.

2. Verfahren nach Anspruch 1, wobei die Medikamentenprobe einer Verschreibung für den Patienten entspricht.

3. Verfahren nach Anspruch 1, wobei der Identifizierungsschritt ferner die Identifizierung der Komponenten, der Konzentration und der Dosierung der Medikamentenprobe beinhaltet.

4. Verfahren nach Anspruch 1, wobei der Satz der Datenbanken beinhaltet:
a.) eine Verschreibungsdatenbank;
b.) eine Allergiedatenbank; und
c.) eine Medikamentenwechselwirkungsdatenbank.

5. Verfahren nach Anspruch 1, wobei die Zuführungsverhinderungseinheit irgendeine von den nachstehenden ist:
a.) ein Durchflussventil; und
b.) eine Absperreinrichtung einer IV-Infusionsleitung.

6. Verfahren nach Anspruch 1, wobei die Medikamentenquelle eine volumetrische Pumpe ist.

7. Verfahren nach Anspruch 1, wobei die Medikamentenquelle eine Spritzenpumpe ist.

8. Verfahren nach Anspruch 1, wobei die Medikamentenquelle eine manuell betätigte Infusionsvorrichtung ist.

## Revendications

1. Procédé d'identification de médicament avant administration, le procédé comprenant :
a) la réception (220) d'un échantillon de médicament à partir d'une source de médicament dans une unité d'identification de médicament ;
b) l'identification (230) de l'échantillon de médicament avec l'unité d'identification de médicament ;
c) l'établissement des correspondances (240) entre l'échantillon de médicament identifié et un ensemble de bases de données, de telle sorte que l'ensemble de bases de données est configuré de manière à confirmer que l'échantillon de médicament identifié est approprié et/ou sûr pour un patient ; et
d) l'interdiction (280) d'administration de l'échantillon de médicament identifié à un patient avec une unité d'interdiction d'administration (160) lorsque l'ensemble de bases de données ne confirme pas que l'échantillon de médicament identifié est approprié et/ou sûr pour un patient.

2. Procédé selon la revendication 1, dans lequel l'échantillon de médicament correspond à une prescription pour le patient.

3. Procédé selon la revendication 1, dans lequel l'étape d'identification comporte, en outre, l'identification des composants, de la concentration et du dosage de l'échantillon de médicament.

4. Procédé selon la revendication 1, dans lequel l'ensemble de bases de données comporte :
a) une base de données de prescription ;
b) une base de données d'allergie ; et
c) une base de données d'interaction de médicament.

5. Procédé selon la revendication 1, dans lequel l'unité d'interdiction d'administration est l'un quelconque des éléments suivants :
a) une vanne d'écoulement ; et
b) un dispositif de restriction de ligne IV.

6. Procédé selon la revendication 1, dans lequel la source de médicament est une pompe volumétrique.

7. Procédé selon la revendication 1, dans lequel la source de médicament est une pompe à seringue.

8. Procédé selon la revendication 1, dans lequel la source de médicament est un dispositif de perfusion manuel.
